(19) Europäisches Patentamt
European Patent Office
Office européen des brevets

(11) Veröffentlichungsnummer: **0 150 769**
**B1**

(12) # EUROPÄISCHE PATENTSCHRIFT

(45) Veröffentlichungstag der Patentschrift: **11.05.88**

(51) Int. Cl.⁴: **C 07 C 127/24**

(21) Anmeldenummer: **85100425.9**

(22) Anmeldetag: **17.01.85**

(54) **Verfahren zur Herstellung von Polyisocyanaten mit Biuretstruktur.**

(30) Priorität: **31.01.84 DE 3403277**

(43) Veröffentlichungstag der Anmeldung: **07.08.85 Patentblatt 85/32**

(45) Bekanntmachung des Hinweises auf die Patenterteilung: **11.05.88 Patentblatt 88/19**

(84) Benannte Vertragsstaaten: **BE DE FR GB IT NL**

(56) Entgegenhaltungen: **EP - A - 0 051 211**

(73) Patentinhaber: **BAYER AG, Konzernverwaltung RP Patentabteilung, D-5090 Leverkusen 1 Bayerwerk (DE)**

(72) Erfinder: **König, Klaus, Dr., Heymannstrasse 50, D-5090 Leverkusen 1 (DE)**
Erfinder: **Pedain, Josef, Dr., Haferkamp 6, D-5000 Köln 80 (DE)**
Erfinder: **Woynar, Helmut, Dr., Ahornweg 7, D-4047 Dormagen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein verbessertes Verfahren zur Herstellung von Polyisocyanaten mit Biuretstruktur, die ausgezeichnete Farbqualität und gute Monomerenstabilität aufweisen, durch Umsetzung von aliphatischen Diisocyanaten mit bestimmten Carbonsäuren als Biuretisierungsmittel.

Aliphatische Polyisocyanate mit Biuretstruktur, insbesondere auf der Basis von Hexamethylendiisocyanat, haben weltweit für die Herstellung lichtechter und extrem witterungsbeständiger Lacke mit höchster Glanzhaltung technische Bedeutung erlangt. Für den Einsatz auf diesem Gebiet, insbesondere für klare und weiss-pigmentierte Beschichtungen, werden vom Markt wenig bis nicht gefärbte Produkte verlangt. Weiterhin ist für eine gefahrlose Verarbeitung ein möglichst geringer Anteil an monomeren Diisocyanaten anzustreben, der sich auch bei längerer Lagerung nicht erhöht. Aufgrund toxikologischer Untersuchung ist eine gefahrlose Verarbeitung bis zu einem maximalen Gehalt von 0,7% an monomerem Diisocyanat gegeben, sofern die bei der Lackverarbeitung üblichen Schutzvorschriften eingehalten werden. Der vorgenannte Grenzwert hat in die Literatur (z.B. Merkblatt «PUR-Anstrichstoffe» des Hauptverbandes der deutschen gewerblichen Berufsgenossenschaft sowie «Polyurethane Report» der Paintmakers Association) Eingang gefunden.

Für die Herstellung solcher Polyisocyanate ist im Laufe der Zeit eine Vielzahl von Verfahren bekannt geworden, die jedoch alle mit speziellen Problemen und Nachteilen behaftet sind und die obengenannte Forderungen an das Produkt nicht oder nur unvollständig erfüllen. Beispielsweise sind folgende Verfahren beschrieben:

– Synthese aus Diisocyanaten und Wasser, gegebenenfalls in Gegenwart von Katalysatoren; siehe DE-PS 1 110 394, DE-OS 1 668 377, DE-OS 2 308 015; GB-PS 889 050, GB-PS 1 399 228, DDR-PS 140 744

– Synthese aus Diisocyanaten und Wasser in Gegenwart eines Lösungsmittels, bzw. eines Lösungsmittelgemisches, siehe DE-OS 2 808 801, DE-OS 3 030 655

– Synthese aus Diisocyanaten und Wasser, wobei das Wasser dampfförmig zur Reaktion gebracht wird, siehe DE-OS 2 918 739

– Synthese aus Diisocyanaten und Ammoniak, bzw. Ammoniak-Wasser-Gemischen, gegebenenfalls in Anwesenheit von Katalysatoren, siehe DE-AS 1 227 003

– Synthese aus Diisocyanaten und Aminen; siehe DE-PS 1 165 580, DE-PS 1 174 759, DE-OS 1 568 017, DE-OS 1 963 190, DE-OS 2 010 887, DE-OS 2 261 065, DE-AS 2 438 258, US-P 3 824 266, DE-AS 2 609 995, DE-OS 2 803 103, DE-PS 883 504, GB-PS 1 263 609, siehe auch Angew. Chem. 72, S. 1002.

– Synthese aus Diisocyanaten und Amin/Alkohol-Gemischen; siehe DE-OS 2 654 745

– Synthese aus Diisocyanaten und ω,ω'-Diaminopolyethern; siehe DE-OS 1 570 632, DE-AS 1 215 365

–Synthese aus Diisocyanaten und substituierten Harnstoffen; siehe DE-PS 1 101 394; DE-AS 1 227 004

– Synthese aus Diisocyanaten und tertiären Alkoholen, gegebenenfalls in Anwesenheit von Katalysatoren; siehe DE-AS 1 543 178, DE-AS 1 931 055, DE-OS 2 308 015

– Synthese aus Diisocyanaten und Ameisensäure (DE-PS 1 174 760; DE-OS 2 308 015)

– Synthese aus Diisocyanaten und Aldoximen (DE-OS 3 007 679)

Verfahren, bei denen die Diisocyanate mit Wasser zur Reaktion gebracht werden, sind infolge der Inhomogenität des Reaktionsgemisches schwer zu beherrschen. Zum einen kommt es zur Ausbildung von äusserst schwerlöslichem Polyharnstoff, zu dessen Auflösung hohe Temperaturen über einen langen Zeitraum notwendig sind und wodurch die Farbe des Produktes negativ beeinflusst wird. Selbst dann bleibt u.U. ein Teil dieser Polyharnstoffe als schwerfiltrierbarer Niederschlag ungelöst und muss vor der Weiterverarbeitung aufwendig abgetrennt werden. Weiterhin können sich infolge der Wasserdampfflüchtigkeit der meisten Diisocyanate Harnstoffablagerungen im Dampfraum des Reaktionsgefässes bilden. Dies gilt auch für Verfahren, bei denen Wasser dampfförmig eingesetzt wird.

Diese Ablagerungen können bei Verwendung von Wasser als Biuretisierungsmittel nur dann vermieden werden, wenn Lösungsmittel bzw. Lösungsmittelgemische zur Homogenisierung des Reaktionsgemisches eingesetzt werden. Diese Verfahren sind jedoch mit verschiedenen Nachteilen verbunden. Zum einen sind grosse Lösungsmittelmengen notwendig, die in einem weiteren Verfahrensschritt dem fertigen Produkt durch Destillation entzogen werden müssen, zum anderen sind für farblose Produkte ganz spezielle Lösungsmittelgemische aus Glykoletheracetaten und Phosphorsäureestern notwendig. Weiterhin sind bei diesen Verfahren Reaktionstemperaturen von mindestens 140 °C notwendig, um das intermediäre Ausfällen von unlöslichen Harnstoffen zu vermeiden. Bilden sich dennoch, z.B. infolge niedrigerer Reaktionstemperaturen, solche Ausfällungen, sind, wie auch bei den Verfahren, bei denen Wasser ohne Lösungsmittel eingesetzt wird, Temperaturen von 160 °C und mehr notwendig, um klare Produkte zu erhalten. Diese Temperaturbelastung führt zu einem vermehrten Auftreten von Nebenreaktionen sowie zu einer deutlichen Verschlechterung der Farbqualität.

Ohne Lösungsmittel können Verfahren durchgeführt werden, bei denen Wasser während der Reaktion aus einer wasserabspaltenden Verbindung freigesetzt wird. Hierzu gehört insbesondere auch das technisch bedeutsame Verfahren mit tert.-Butanol als Biuretisierungsmittel. Dieses Verfahren erfordert jedoch ebenfalls Temperaturen von ca. 180 °C mit allen bereits angesprochenen Nachteilen für die Qualität des Produktes. Weiterhin beinhaltet dieses Verfahren den Verlust

des Biuretisierungsmittel unter Freisetzung brennbarer Gase (Isobuten).

Die Umsetzung von Diisocyanaten mit Aldoximen ist ebenfalls gekennzeichnet durch den Verlust des schwer zugänglichen Biuretisierungsmittels und das Auftreten von leichtflüchtigen Nebenprodukten (Nitrile), die nicht wiederverwendet werden können.

Die Reaktion von Diisocyanaten mit Schwefelwasserstoffen liefert das giftige, leichtsiedende Kohlenoxisulfid, das ebenfalls nicht wieder in den Prozess zurückgeführt werden kann und aufwendig entsorgt werden muss.

Allen bisher genannten Verfahren ist gemeinsam, dass ein Teil des Diisocyanates durch Reaktion mit dem Biuretisierungsmittel in Amine, also die Vorstufe der Isocyanate überführt wird. Es wurden deshalb Verfahren vorgeschlagen, Diisocyanate direkt mit Aminen zu den Biuret-Polyisocyanaten umzusetzen.

Dabei werden jedoch auch bei Einsatz hochentwickelter Mischverfahren infolge der hohen Reaktivität der Amine schwerlösliche Polyharnstoffe erhalten, zu deren Auflösung eine starke Temperaturbelastung notwendig ist, verbunden mit einer Verschlechterung der Farbqualität sowie dem vermehrten Auftreten von Nebenprodukten. Neben Uretdionen und Isocyanuraten treten dabei auch Carbodiimide bzw. Folgeprodukte von Carbodiimiden auf, die die Monomerenstabilität des Endproduktes ungünstig beeinflussen.

Herabgesetzt werden kann die Tendenz zur Bildung von schwerlöslichen Polyharnstoffen durch Einsatz von Diaminen, die auf geeignete Weise in ihrer Reaktivität deutlich herabgesetzt sind, z.B. durch sterische Hinderung. Die Produkte enthalten u.a. jedoch einen hohen Anteil an monomeren Diisocyanaten, die aus den eingesetzten Diaminen entstehen, und die nicht durch Dünnschichtdestillation zu entfernen sind.

Beim Einsatz von ω,ω'-Diaminopolyethern werden wohl flüssige, biurethaltige Polyisocyanate erhalten, diese Lösung ist jedoch wegen der zusätzlichen Synthese des Biuretisierungsmittels sehr aufwendig. Ausserdem führen die in diesen Produkten vorhandenen Ethergruppierungen zu einem schlechten Bewitterungsverhalten daraus hergestellter Lackfilme.

Vermieden werden kann das Auftreten von Polyharnstoffen durch Einsatz von Monoaminen oder N,N'-disubstituierten Harnstoffen. Hierbei müssen jedoch die aus diesen Biuretisierungsmittel resultierenden, leichtflüchtigen Monoisocyanate aus dem Reaktionsgemisch entfernt werden. Dies ist jedoch selbst bei hoher Temperatur wegen der notwendigen Equilibrierungsreaktion nur unvollständig möglich.

Bei der Umsetzung von Diisocyanaten mit Ameisensäure lassen sich zwar unter schonenden Bedingungen Produkte mit guter Farbqualität herstellen, die jedoch noch in hohem Ausmass N-Formylgruppierungen enthalten. Um ein Polyisocyanat mit überwiegender Biuretstruktur zu erhalten, sind Reaktionstemperaturen von mehr als 160 °C über längere Zeit nötig, was zu einer deutlichen Gelbfärbung der Produkte führt. Das Biuretisierungsmittel wird darüber hinaus verbraucht unter Freisetzung von giftigem Kohlenmonoxid, wodurch in verstärktem Masse Abluftprobleme hervorgerufen werden.

Ebenfalls vorgeschlagen wurden Verfahren, bei denen Diisocyanate und Amin-Alkohol-Gemische eingesetzt werden. Abgesehen von anderen Nachteilen resultieren aus solchen Verfahrensweisen Produkte veränderter Struktur mit unterschiedlichem Eigenschaftsbild. Das gleiche gilt für Produkte, bei deren Herstellung Diisocyanate mit Ammoniak umgesetzt werden.

Es wurde nun gefunden, dass man Polyisocyanate mit Biuretstruktur in ausgezeichneter Farbqualität und mit guter Monomerenstabilität durch Umsetzung von aliphatischen Diisocyanaten mit bestimmten, nachstehend näher beschriebenen Monocarbonsäuren unter Einhaltung bestimmter Mengenverhältnisse der Reaktionspartner bei vergleichsweise niedrigen Temperaturen herstellen kann.

Gegenstand der Erfindung ist somit ein verbessertes Verfahren zur Herstellung von Polyisocyanaten mit Biuretstruktur durch Umsetzung von überschüssigen Mengen an aliphatischen Diisocyanaten mit Biuretisierungsmitteln bei erhöhter Temperatur, gegebenenfalls unter Überdruck, wobei die Umsetzung gegebenenfalls in Gegenwart von mit Wasser mischbaren Lösungsmitteln erfolgt, und wobei gegebenenfalls im Anschluss an die Biuretisierungsreaktion überschüssige Mengen an nicht umgesetztem Ausgangsdiisocyanat, sowie Lösungsmittel und sonstige flüchtige Bestandteile des Reaktionsgemisches durch Destillation und/oder Extraktion von dem entstandenen Biuretpolyisocyanat abgetrennt werden, dadurch gekennzeichnet, dass man als Biuretisierungsmittel

a) α, α,α-trisubstituierte Essigsäuren, welche neben der Carboxylgruppe keine gegenüber Isocyanatgruppen reaktionsfähige Gruppen auf weisen und gegebenenfalls

b) Wasser verwendet, wobei ein Molverhältnis der Biuretisierungsmittelkomponenten a) zu b) von 1:0 bis 1:2,5 eingehalten wird.

Für das erfindungsgemässe Verfahren werden lineare oder verzweigte aliphatische Diisocyanate mit 4–30, vorzugsweise 5–12 Kohlenstoffatomen im Kohlenwasserstoffrest, die gegebenenfalls auch eine oder mehrere Estergruppierungen aufweisen können, verwendet.

Genannt seien beispielsweise: 1,4-Diisocyanatobutan,1,5-Diisocyanato-pentan, 1,6-Diisocyanatohexan, 1,8-Diisocyanato-octan, 1,10-Diisocyanato-decan, Isomerengemisch aus 2,2,4-Trimethyl-1,6-diisocyanatohexan und 2,4,4,-Trimethyl-1,6-diisocyanatohexan, 2-Methyl-1,5-diisocyanato-pentant, 2,2-Dimethyldiisocyanato-pentan, 6-Isocyanato-hexansäure-(2-isocyanatoethyl)-ester, 2,6-Bis-(isocyanato)-hexansäuremethylester. Besonders bevorzugt wird 1,6-Diisocyanatohexan als Ausgangsdiisocyanat eingesetzt. Die Ausgangsdiisocynate können in handelsüblicher Qualität eingesetzt werden. d.h. eine Vorreinigung oder Temperung ist nicht erforderlich.

Unter «Biuretisierungsmittel» sind erfindungsgemäss organische Verbindungen zu verstehen, die bei erhöhter Temperatur mit Isocyanatgruppen unter Biuretbildung reagieren. So ist beispielsweise tert.-Butanol ein bekanntes Biuretisierungsmittel des Standes der Technik. Erfindungswesentlich ist nun die Verwendung von a) bestimmten trisubstituierten Essigsäuren, gegebenenfalls in Kombination mit b) Wasser als Biuretisierungsmittel.

Als Biuretisierungsmittelkomponente a) geeignete α,α,α-trisubstituierte Essigsäuren sind beliebige trisubstituierte Essigsäuren, die am α-Kohlenstoffatom keinen Wasserstoff gebunden enthalten, und die neben der Carboxylgruppe keine gegenüber Isocyanatgruppen reaktionsfähige Gruppe aufweisen. Beispiele geeigneter α, α,α-trisubstituierter Essigsäuren sind Verbindungen der allgemeinen Formel

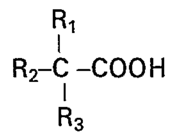

in der $R_1$, $R_2$ und $R_3$ für gleiche oder verschiedene Reste stehen und Alkyl-, Alkoxy- oder Alkoxyalkyl-Gruppen bedeuten, wobei gegebenenfalls zwei der Reste zusammen mit dem substituierten Kohlenstoffatom, der Essigsäure einen cycloaliphatischen Ring bilden, und wobei vorzugsweise die Summe der Anzahl der in den Resten $R_1$, $R_2$ und $R_3$ vorliegenden Kohlenstoffatome 3 bis 6 beträgt.

Geeignete Säuren sind beispielsweise 2,2-Dimethylbuttersäure; 2,2,3-Trimethylbuttersäure; 2-Methyl-2-methoxymethylpropionsäure; 1-Methylcyclopropancarbonsäure und insbesondere Trimethylessigsäure (Pivalinsäure). Auch Methacrylsäure stellt eine erfindungsgemäss geeignete α α,α-trisubstituierte Essigsäure dar, da sie der obengenannten Bedingung, dass das α-Kohlenstoffatom der Essigsäure mit keinem Wasserstoffatom verknüpft ist, genügt.

Die erfindungsgemäss einzusetzenden Säuren sollten im Interesse einer wirtschaftlichen Durchführbarkeit des Verfahrens vorteilhafterweise bestimmte Voraussetzungen erfüllen. Sie sollten zum einen zugänglich sein, zum anderen sollten die aus ihnen entstehenden Anhydride eine genügend grosse Siedepunktdifferenz zum eingesetzten Diisocyanat aufweisen, um noch vor einer durchzuführenden Dünnschichtverdampfung aus dem Reaktionsgemisch destillativ abgetrennt werden zu können. Wird als Isocyanatkomponente das bevorzugte 1,6-Diisocyanatohexan eingesetzt, so ist z.B. letztere Forderung für Pivalinsäure und 2,2-Dimethylbuttersäure erfüllt, während die Anhydride anderer α,α,α-trisubstituierter Essigsäuren gleich hoch oder höher sieden als 1,6-Diisocyanatohexan und deshalb, wenn sie abgetrennt und nach Hydrolyse wieder eingesetzt werden sollen, einen zusätzlichen Destillationsvorgang nach der gemeinsamen Abtrennung von überschüssigem Diisocyanat und Säureanhydrid vom Reaktionsgemisch durch Dünnschichtverdampfung erfordern würden. Da Pivalinsäure darüber hinaus technisch verfügbar ist, wird sie für das erfindungsgemässe Verfahren bevorzugt eingesetzt.

Bei der Durchführung des erfindungsgemässen Verfahrens können die beispielhaft genannten α,α,α-trisubstituierten Essigsäuren als alleiniges Biuretisierungsmittel eingesetzt werden. Gemäss einer zweiten Variante des erfindungsgemässen Verfahrens gelangen die erfindungswesentlichen Biuretisierungsmittel in Kombination mit Wasser zum Einsatz, so dass die Kombination aus substituierter Essigsäure und Wasser das Biuretisierungsmittel darstellt. Im allgemeinen werden die Biuretisierungsmittel a) und gegebenenfalls b) in einem Molverhältnis Säure:Wasser von 1:0 bis 1:2,5, vorzugsweise von 1:0 bis 1:1,5 entsprechenden Mengen eingesetzt. Die Gesamtmenge des eingesetzten Biuretisierungsmittels entspricht hierbei einem Molverhältnis von Ausgangsdiisocyanat: «Gesamtwasser» von 3:1 bis 20:1, vorzugsweise von 5:1 bis 12:1, wobei unter «Gesamtwasser» in diesem Zusammenhang sowohl das gegebenenfalls als Biuretisierungsmittelkomponente b) zugesetzte Wasser als auch das Kondensationswasser zu verstehen ist, welches theoretisch bei quantitativer Überführung der eingesetzten trisubstituierten Essigsäure in ihr Anhydrid unter Wasserabspaltung entstehen würde. Die Angabe «1 Mol Wasser» entspricht somit, bezogen auf die, formal als Wasser abspaltende Verbindung anzusehenden, trisubstituierten Essigsäuren, der Angabe «2 Mol trisubstituierte Essigsäure».

Grundsätzlich wäre es auch denkbar, zusätzlich zu den erfindungsgemäss einzusetzenden Biuretisierungsmitteln weitere Biuretisierungsmittel der an sich bekannten Art mitzuverwenden. Dies würde jedoch zu keinerlei Verbesserung des Verfahrens bzw. der Verfahrensprodukte führen.

Es kann zweckmässig sein, insbesondere bei Mitverwendung von Wasser, das erfindungsgemässe Verfahren in Gegenwart eines mit Wasser zumindest in gewissen Grenzen mischbaren, gegen Isocyanate und Säuren inerten Lösungsmittels durchzuführen. Als gegebenenfalls mitzuverwendende Lösungsmittel seien beispielhaft benannt: Ether, wie Di-iso-propylether, Ethylenglykoldimethylether, Diethylenglykoldimethylether, 1,4-Dioxan, Tetrahydrofuran, 1,2-Dimethoxypropan; Ester, wie Butyrolacton, Ethylenglykolcarbonat, Propylenglykolcarbonat; Ether-Ester, wie Methoxyethylacetat, Ethoxyethylacetat, 1-Methoxypropyl-2-acetat, 2-Methoxypropyl-1-acetat; 1-Ethoxypropyl-2-acetat, 2 Ethoxypropyl-1-acetat; Ketone, wie Aceton, Methylethylketon; Nitrile, wie Acetonitril, Propionitril, Methoxypropionitril Sulfone, wie Sulfolan, Dimethylsulfon, Diethylsulfon; Phosphorsäureester, wie Triethyl-/Trimethylphosphat.

Als weniger bevorzugte Lösungsmittel seien genannt: Tetramethylharnstoff, N-Methylpyrrolidon, Dimethylformamid, Dimethylacetamid.

Das erfindungsgemässe Verfahren wird bei Temperaturen von 20–160 °C, vorzugsweise 60–140 °C durchgeführt.

Üblicherweise wird das Verfahren bei Normaldruck durchgeführt. Insbesondere bei Mitverwendung von Wasser und/oder niedrigsiedenden Lösungsmitteln kann das Verfahren natürlich auch bei Drücken von 1–50 bar, vorzugsweise 1–5 bar durchgeführt werden.

Als Hauptprodukt der Reaktion von Isocyanaten mit Carbonsäure wird in der Literatur normalerweise das Carbonsäureamid beschrieben.

$$R{-}NCO + R'{-}COOH \rightarrow R{-}NH{-}\overset{O}{\overset{\|}{C}}{-}R' + CO_2$$

Nur an einigen Stellen wird die intermediäre Bildung von gemischten Anhydriden beschrieben, die thermisch unbeständig sind und unter Kohlendioxidabspaltung zu Amid zerfallen. Im Falle einiger weniger Isocyanate wurde auch über einen anderen Zerfallsweg berichtet, wobei das gebildete Anhydrid teilweise unter Bildung von Harnstoff und Carbonsäureanhydrid zerfällt.

$$2\,R{-}NCO + 2R'COOH \rightarrow 2R{-}NH{-}\overset{O}{\overset{\|}{C}}{-}O{-}\overset{O}{\overset{\|}{C}}{-}R$$

$$\text{I}\quad 2\,R{-}NH{-}\overset{O}{\overset{\|}{C}}{-}R' + 2\,CO_2$$

$$\text{II}\quad R{-}NH{-}\overset{O}{\overset{\|}{C}}{-}NH{-}R + R'{-}\overset{O}{\overset{\|}{C}}{-}O{-}\overset{O}{\overset{\|}{C}}{-}R' + CO_2$$

Bei Einsatz aliphatischer Diisocyanate wird jedoch überwiegend Zerfall in das Carbonsäureamid festgestellt.

Der nach II gebildete Harnstoff kann bei Vorliegen von überschüssigem Isocyanat mit diesem zu Biureten weiterreagieren oder vom gebildeten Anhydrid acyliert werden.

Zwar wird in der DE-AS 1 174 760 beschrieben, dass im Falle des Einsatzes von Ameisensäure als Carbonsäure die ausschliessliche Bildung von Biureten unter Abspaltung von Kohlenmonoxid als Anhydrid der Ameisensäure erfolgt. Eigene Nacharbeiten sowie die Lehre der DE-OS 2 308 015 zeigen jedoch, dass in den Produkten entsprechend der DE-AS 1 174 760 in sehr hohem Ausmass N-Formylharnstoff-Gruppierungen anstelle von Biuret-Gruppierungen vorliegen müssen. Ein weiterer Hinweis ist das Beispiel 1 der DE-AS 1 174 760 (dort Spalte 3, Zeile 51 bis Spalte 4, Zeile 10). Sowohl der gefundene NCO-Gehalt als auch die Viskosität von 853 mPas bei 25 °C des dort beschriebenen Produktes zeigen eindeutig, dass dort in hohem Masse N-Formylharnstoffe vorliegen. Ein Polyisocyanat mit ausschliesslich Biuretstruktur müsste bei der beschriebenen Zusammensetzung des Reaktionsgemisches einen NCO-Gehalt von ca. 22,8% und eine Viskosität von ca. 7000 mPas bei 25 °C aufweisen.

Eigene Versuche (siehe Vergleichsbeispiel I) bestätigen dies in eindeutiger Weise. Die Produktzusammensetzung wurde mit Hilfe der Gelchromatographie überprüft und es wurde gefunden, dass das Verhältnis von Biuretgruppen zu Formylharnstoffen in den Produkten je nach Reaktionsbedingungen ca. 1:3 bis 3:1 beträgt, wobei die Produkte mit grösseren Mengen an Biuretgruppen zusätzlich in beträchtlichem Ausmass Nebenprodukte wie Uretdione enthalten und darüber hinaus stark gefärbt sind.

Überraschenderweise wurde jetzt gefunden, dass überschüssige aliphatische Diisocyanate der genannten Art, wenn man sie mit α,α, α-trisubstituierten Essigsäuren umsetzt, nahezu ausschliesslich unter Bildung von Carbonsäureanhydriden und intermediären Harnstoffbildung zu Polyisocyanaten mit Biuretstruktur reagieren, wobei dieser intermediär gebildete Harnstoff in Gegenwart von überschüssigem Diisocyanat rasch und quantitativ weiterreagiert bei Temperaturen, bei denen N,N'-dialkylsubstituierte Harnstoffe normalerweise noch nicht oder sehr viel langsamer mit Isocyanaten reagieren.

Des weiteren wurde überraschend gefunden, dass in Gegenwart der erfindungsgemässen Säuren die Bildung von Biureten aus Diisocyanaten und Wasser sehr viel rascher und bei niedrigeren Temperaturen abläuft als in Abwesenheit dieser Säuren. Offenbar wirkt hierbei die Säure als Überträger, in dem das eingesetzte Wasser das gebildete Säureanhydrid hydrolysiert und so die Säure zurückgebildet wird.

Ameisensäure kann nicht im beschriebenen Sinne als Überträger wirken, da sie Kohlenmonoxid als inneres Anhydrid bildet, aus dem unter den Reaktionsbedingungen natürlich nicht mit Wasser Ameisensäure zurückgebildet werden kann.

Andere Säuren wie z.B. Essigsäure können die beschriebene Wirksamkeit nur in begrenztem Masse ausüben, da sie zum überwiegenden Teil unter Kohlendioxidabspaltung zu unter den Reaktionsbedingungen ebenfalls nicht hydrolysierbaren Carbonsäureamiden reagieren und somit schnell aus dem Reaktionsgemisch verschwinden.

Wegen der Rückbildung der erfindungswesentlichen Säuren aus Wasser und Anhydrid ist das erfindungsgemässe Verfahren auch dann noch möglich, wenn die Biuretbildung formal zum Teil durch α,α,α-trisubstituierte Essigsäuren und zum Teil durch Wasser erfolgt. Zur Herstellung von Polyisocyanaten mit Biuretstruktur können erfindungsgemäss somit Gemische der Säuren mit Wasser als Biuretisierungsmittel eingesetzt werden.

Das erfindungsgemässe Verfahren wird beispielsweise wie folgt durchgeführt:

Das einzusetzende Diisocyanat wird bei Raumtemperatur in einem Rührreaktor, der gegebenenfalls mit einer Messeinrichtung für das entstehende Kohlendioxid versehen ist, vorgelegt.

Die Säure wird aus einer Vorlage, die gegebenenfalls beheizbar ist, um bei Raumtemperatur feste Säuren in geschmolzener Form einsetzen zu können, im Verlauf von ca. 30 min. in das vorgelegte Diisocyanat eindosiert, wobei die exotherme Bildung des gemischten Anhydrids aus Isocyanat und Säure unter Erwärmung des Reaktionsgemisches auf etwa 50 °C einsetzt. Bei dieser Temperatur beginnt bereits eine mässige Gasentwicklung, die durch Aufheizen auf 60–80 °C mit einer vertretbaren Geschwindigkeit weitergeführt wird. Der gegen Ende der Reaktion einsetzende Verlangsamung der Gasentwicklung begegnet man durch Erwärmen auf 100–120 °C. Der Zeitbedarf für die gesamte Reaktion beträgt 2–4 Stunden.

Die Reaktion kann natürlich auch bei niedrigeren Temperaturen durchgeführt werden, z.B. bei Raumtemperatur, wenn die Reaktionswärme der Primärreaktion zwischen Diisocyanat und Wasser auf geeignete Weise abgeführt wird. Sie dauert dann bis zur Vollständigkeit mehrere Tage. Jedoch treten bei dieser Art der Temperaturführung anfänglich Trübungen auf, die durch anschliessende längere Wärmebehandlung bei 120–140 °C wieder in Lösung gebracht werden müssen.

Selbstverständlich kann man auch das Diisocyanat, gegebenenfalls im Gemisch mit einem Lösungsmittel, bereits bei erhöhter Temperatur von 80–120 °C vorlegen und Säure und gegebenenfalls Wasser in dem Mass zudosieren, wie die Reaktion fortschreitet. Diese Verfahrensweise würde vorteilhaft in solchen Fällen angewandt, bei denen Wasser mitverwendet wird, und zwar sollte zur Vermeidung von schwerlöslichen Harnstoffausfällungen die Anfangstemperatur um so höher gewählt werden, je höher der Wasseranteil im Biuretisierungsmittel ist.

Bei ausschliesslicher Verwendung der erfindungsgemässen Säuren als Biuretisierungsmittel sind die Reaktionsgemische weitgehend homogen, die Mitverwendung eines Lösungsmittels ist normalerweise nicht erforderlich und bringt keine Vorteile. Wird jedoch ein Teil der Biuretgruppen durch Reaktion des Diisocyanats mit Wasser erzeugt, kann das entweichende Kohlendioxid Wasser mitreissen, das dann an den oberen Teilen des Reaktionsgefässes kondensiert oder in die Abluft gelangt und somit der Reaktion entzogen wird. Durch Zugabe eines Lösungsmittels mit einem geeigneten Siedepunkt, das mit Wasser zumindest in gewissen Grenzen mischbar ist, können diese unkontrollierten Wasserverluste vermieden werden.

Ein weiterer Vorteil der Mitverwendung eines Lösungsmittels besteht darin, dass bei Mitverwendung von Wasser ein homogeneres Reaktionsgemisch enthalten wird, wodurch sich die Reaktionsgeschwindigkeit erhöht. Die für diesen Zweck notwendigen Lösungsmittelmengen sind deutlich niedriger als z.B. bei dem Verfahren entsprechend der DE-OS 2 808 801, üblicherweise werden Mengen eingesetzt, die 3–20 Gew.-% des eingesetzten Diisocyanats entsprechen. Die Mengen können insbesondere auch deshalb so gering gehalten werden, da ein vollständig homogenes Reaktionsgemisch, wie es für das Verfahren der DE-OS 2 808 801 vorliegen muss, wegen der alternativen Reaktionsweise des erfindungsgemässen Verfahrens nicht notwendig ist.

Das gegebenenfalls mitzuverwendende Lösungsmittel kann ganz oder teilweise zusammen mit dem Diisocyanat vorgelegt werden oder ganz oder teilweise mit der Säure bzw. dem gegebenenfalls mitzuverwendenden Wasser dem Diisocyanat zudosiert werden. Säure und Wasser können natürlich entweder separat oder zusammen, gegebenenfalls im Gemisch mit Lösungsmitteln, eingesetzt werden.

Im Falle der Mitverwendung von Wasser und/oder Lösungsmitteln kann es zweckmässig sein, zur Vermeidung von Verlusten dieser Komponenten unter Überdruck zu arbeiten. Vorteilhaft wird dabei der maximale Druck während der Reaktion durch geeignete Massnahmen z.B. durch ein Druckhalterventil, auf 6 bar begrenzt, da bis zu diesem Druck die Benutzung üblicher technischer Apparate problemlos möglich ist. Die Reaktion kann selbstverständlich auch unter höheren Drücken, z.B. unter dem vollständigen Eigendruck des bei der Reaktion entstehenden Kohlendioxids durchgeführt werden, wobei je nach Art der Temperaturführung und Befüllungsgrad des Reaktors Drücke bis zu maximal 20 bar auftreten können. In solchen Fällen sind jedoch spezielle Hochdruckapparaturen notwendig.

Das Verhältnis von NCO-Gruppen zu Säure und Wasser kann, wie bereits ausgeführt, in weiten Grenzen variiert werden. Es bestimmt die Oligomerenverteilung des resultierenden Biuret-Polyisocyanats und damit massgebliche Eigenschaften des Produktes, z.B. Isocyanatgehalt und Viskosität. So weisen zwei handelsübliche Biuretpolyisocyanate Viskositäten von 10 000 bzw. 2 500 mPas bei 23 °C auf. Diese Viskositäten werden beispielsweise erreicht, wenn entsprechend dem erfindungsgemässen Verfahren $^3/_{11}$ bzw. $^1/_6$ der eingesetzten NCO-Gruppen mit Säure oder Säure/Wasser-Gemischen zu Biureten reagieren.

Selbstverständlich können durch grössere Überschüsse an Isocyanat Endprodukte noch geringerer Viskosität hergestellt werden. Das Verfahren wird jedoch infolge des höheren Destillationsaufwandes, der geringeren Raum-Zeit-Ausbeute und des vermehrten Auftretens von Nebenprodukten schnell unökonomisch. Zur anderen Seite wird die Variationsbreite begrenzt durch das Auftreten von Produkten mit extrem hohen Viskositäten, die zunehmend mit unpolaren Lösungsmitteln, wie sie für die Verarbeitung von Polyisocyanaten eingesetzt werden, unverträglicher werden. Bei Umsetzung von mehr als der Hälfte der NCO-Gruppen des ursprünglich vorliegenden Diisocyanates muss natürlich mit der Bildung unlöslicher Gele gerechnet werden.

Nach Beendigung der Reaktion werden überschüssiges Diisocyanat, Carbonsäureanhydride und gegebenenfalls vorhandenes Lösungsmittel

aus dem Reaktionsgemisch destillativ entfernt. Wurde kein Lösungsmittel verwendet, ist es vorteilhaft, das gebildete Säureanhydrid, wenn es niedriger siedet als das eingesetzte Diisocyanat, zunächst durch Destillation im Vakuum dem Reaktionsgemisch weitestgehend zu entziehen. In der Praxis können ohne grösseren Aufwand etwa 90% des im Reaktionsgemisch vorhandenen Anhydrids in reiner Form ohne Anteile an Diisocyanat isoliert werden. Das restiche Anhydrid verbleibt somit im Reaktionsgemisch und wird in einer anschliessenden Dünnschichtdestillation zusammen mit dem überschüssigen Diisocyanat vom Biuret-Polyisocyanat abgetrennt. Da das im überschüssigen Diisocyanat verbleibende Anhydrid für das erfindungsgemässe Verfahren in keiner Weise schädlich ist, kann das erhaltene Gemisch ohne weitere Massnahmen in den Prozess zurückgeführt werden.

Grundsätzlich ist es auch möglich, jedoch weniger bevorzugt, das überschüssige Diisocyanat beispielsweise nach Entfernung des Lösungsmittels und des Säureanhydrids durch Extraktion mit geeigneten Lösungsmitteln wie beispielsweise n-Hexan von dem gebildeten Biurettriisocyanat abzutrennen.

Das vorher abgetrennte Anhydrid kann in einfacher Weise durch Erhitzen mit Wasser zur Säure hydrolysiert und ebenfalls wiederverwendet werden. Da das erfindungsgemässe Verfahren auch mit Gemischen aus Säure und Wasser durchführbar ist, ist eine vollständige Hydrolyse des abgetrennten Anhydrids nicht zwingend notwendig.

Die Verfahrensweise, gebildetes Säureanhydrid vollständig aus der Reaktionslösung destillativ zu entfernen, liefert ein mit Diisocyanat verunreinigtes Produkt, bei dessen Hydrolyse schwerlösliche Carbonsäureamide anfallen. Zur Vermeidung dieser Ausfällungen wäre ein weiterer Destillationsschritt notwendig, diese Verfahrensweise wird daher weniger bevorzugt.

Falls das Anhydrid der eingesetzten Carbonsäure höher siedet, als das eingesetzte Diisocyanat, werden beide zusammen durch Dünnschichtdestillation entfernt und es wird auf jeden Fall ein weiterer Destillationsschritt notwendig, wenn die Säure zurückgewonnen werden soll.

Deshalb wird auch diese Verfahrensweise weniger bevorzugt und es werden vorteilhafterweise solche Säuren eingesetzt, deren Anhydride niedriger sieden als die verwendeten Diisocyanate.

Wird ein Lösungsmittel mitverwendet, kann dieses, je nach Siedepunkt, separat bzw. zusammen mit dem Anhydrid oder zusammen mit dem überschüssigen Diisocyanat aus dem Reaktionsgemisch entfernt und wiederverwendet werden. Wenn es zusammen mit dem gebildeten Säureanhydrid isoliert wird, bedarf es bei der sich anschliessenden Hydrolyse des Anhydrids keiner Abtrennung des Lösungsmittels. Der Einsatz eines Lösungsmittel/Anhydrid-Gemisches zur Hydrolyse kann darüber hinaus vorteilhaft sein, da das Lösungsmittel als Lösungsvermittler zwischen Wasser und dem mit Wasser unter Umständen nicht mischbaren Anhydrid wirkt.

Selbstverständlich eignet sich das Verfahren in hervorragender Weise auch für eine kontinuierliche Durchführung. In diesem Fall wird beispielsweise in den ersten von 4–6 kaskadenförmig hintereinandergeschalteten Rührreaktoren Diisocyanat und Carbonsäure, gegebenenfalls zusammen mit Wasser und/oder einem Lösungsmittel separat oder als Gemisch so zudosiert, dass sich bis zum Verlassen des letzten Reaktors eine Verweilzeit von 2–8 Stunden ergibt. Dabei kann die Temperatur in den einzelnen Reaktoren gleichförmig 100–140 °C betragen oder in den einzelnen Reaktoren ansteigend 60–140 °C, besser 80–120 °C betragen.

Je nach den Siedepunkten von Diisocyanat, gebildetem Säureanhydrid und gegebenenfalls mitverwendeten Lösungsmittel wird das Reaktionsgemisch entweder zuerst über eine kontinuierlich zu betreibende Destillationskolonne zur Abtrennung des Anhydrids, gegebenenfalls zusammen mit dem Lösungsmittel, geleitet und anschliessend das Biuret-Polyisocyanat durch Dünnschichtdestillation oder Extraktion von überschüssigem Diisocyanat sowie Resten von Anhydrid und gegebenenfalls vorhandenem Lösungsmittel befreit, oder zunächst das Biuret-Polyisocyanat durch gemeinsame Dünnschichtverdampfung von überschüssigem Diisocyanat, Anhydrid und Lösungsmittel befreit und das Brüdenprodukt anschliessend über eine Kolonne in Diisocyanat und Anhydrid aufgetrennt, wobei das gegebenenfalls mitverwendete Lösungsmittel entweder separat oder zusammen mit einer der anderen Komponenten abgetrennt werden kann. Das so gewonnene, gegebenenfalls im Gemisch mit Lösungsmittel, vorliegende Anhydrid wird anschliessend kontinuierlich oder diskontinuierlich vollständig oder partiell zur Säure hydrolysiert und, ebenso wie das überschüssige Diisocyanat, in den Prozess zurückgeführt.

Beispielsweise würde bei der bevorzugten Reaktion von 1,6-Diisocyanatohexan mit Pivalinsäure zunächst das gebildete Pivalinsäureanhydrid über eine Kolonne abgetrennt und der Hydrolyse zugeführt und anschliessend das Biuret-Polyisocyanat durch Dünnschichtverdampfung von überschüssigem 1,6-Diisocyanatohexan befreit.

Die nach dem erfindungsgemässen Verfahren hergestellten Polyisocyanate mit Biuret-Struktur zeichnen sich durch hohe Farbqualität sowie gute Lagerstabilität aus und sind weitgehend frei von Nebenprodukten. Sie eignen sich in hervorragender Weise zur Herstellung von lichtechten und extrem witterungsbeständigen Lacken mit höchster Glanzhaltung.

Das erfindungsgemässe Verfahren wird durch die nachfolgenden Beispiele näher erläutert.

Beispiel 1

In einem 10 l-Vierhalskolben mit Kontaktthermometer, Rührer und Rückflusskühler wurden 6 049 g (36 Mol) Hexamethylendiisocyanat bei 20 °C vorgelegt und unter Rühren mit 1 224 g (12 Mol) geschmolzener Pivalinsäure versetzt. Die

Temperatur des Reaktionsgemisches stieg daraufhin spontan auf etwa 50 °C, und es setzte eine schwache $CO_2$-Entwicklung ein. Das Gas wurde am oberen Ende des Kühlers über einen Blasenzähler abgeführt und über eine Gasuhr volumetrisch kontrolliert. Im Verlauf von 2 Stunden wurde die Temperatur auf 80 °C gesteigert und noch eine weitere Stunde auf dieser Höhe belassen. Nach dieser Zeit waren korr. 130 l (5,8 Mol) $CO_2$ freigesetzt worden. Nun wurde zur Beendigung der nachlassenden Reaktion noch 30 Minuten bei 130 °C gerührt. Danach entstand kein $CO_2$ mehr; die Gesamtmenge betrug korr. 136 l (6,07 Mol). Nun wurde auf 80 °C abgekühlt und bei 10 mbar über eine Füllkörperkolonne (50 cm Höhe, 5 cm Durchmesser) Pivalinsäureanhydrid abdestilliert. Eine erste Fraktion von 970 g (87% d.Th.) mit einem Siedepunkt von 81 °C wies eine Reinheit von 99,9% auf. Eine zweite Fraktion (120 g) bestand zu 89 % aus Pivalinsäureanhydrid und zu 11% aus Hexamethylendiisocyanat. Die Gesamtausbeute an Anhydrid betrug also 1 077 g (96,5% d.Th.).

Die 1. Fraktion wurde mit der äquimolaren Menge Wasser 30 Minuten auf 100 °C erhitzt, und die so gewonnene Pivalinsäure ohne weitere Reinigung für nachfolgende Ansätze verwendet.

Das gewonnene rohe Biuretpolyisocyanat war wasserhell und frei von jeglichen Feststoffpartikeln. Die Hälfte des Produktes (2 958 g) wurde durch zweimalige Dünnschichtdestillation (150 °C, 0,5 mbar) von überschüssigem Diisocyanat befreit. Es wurden 1 200 g eines Biuretpolyisocyanats mit folgenden Eigenschaften erhalten:

| | |
|---|---|
| NCO-Gehalt: | 22,5% |
| Viskosität bei 25 °C: | 6 950 mPas |
| APHA-Farbzahl (DIN 53 409): | 30 |
| Gehalt an monomerem Diisocyanat: | 0,3 Gew.-% |
| nach Lagerung (6 Wochen, 50°): | 0,4 Gew.-% |

Die andere Hälfte des Rohproduktes wurde durch fraktionierte Extraktion mit n-Hexan als Lösungsmittel aufgearbeitet:

| | |
|---|---|
| Ausbeute: | 1 150 g |
| NCO-Gehalt: | 22,3% |
| Viskosität bei 25 °C: | 7 500 mPas |
| APHA-Farbzahl: | 20–30 |
| Monomerengehalt: | 0,2 Gew.-% |

Beispiel 2

3 024 g (18 Mol) Hexamethylendiisocyanat wurden bei 120 °C vorgelegt und 408 g (4 Mol) Pivalinsäure innerhalb 2 Stunden zugetropft. Nach einer weiteren Stunde bei der gleichen Temperatur war die $CO_2$-Entwicklung beendet. Es wurden korr. 91 l (4.06 Mol) gemessen. Die Destillation bei 20 mbar Sumpftemperatur 100–140 °C lieferte 378 g die zu 94% aus Pivalinsäureanhydrid bestanden. Ausbeute: 95,5% d.Th. Nach Dünnschichtdestillation wurden 860 g eines Biuretpolyisocyanats mit folgenden Eigenschaften erhalten:

| | |
|---|---|
| NCO-Gehalt: | 23,6% |
| Viskosität bei 25 °C: | 2 540 mPas |
| APHA-Farbzahl: | 40 |
| Monomerengehalt: | 0,15% |
| nach Lagerung (6 Wo, 50°): | 0,23% |

Beispiel 3

2 520 g (15 Mol) Hexamethylendiisocyanat wurden mit 612 g (6 Mol) Pivalinsäure versetzt. Durch ein Wasserbad wurde die Temperatur des Gemisches auf 50 °C eingestellt. Es setzte schwache Gasentwicklung ein; im Verlauf von 24 Stunden entstanden korr. 28,5 l (85% d.Th.) $CO_2$. Es hatte sich ein feinteiliger Niederschlag aus Harnstoff-Zwischenprodukten gebildet. Nun wurde im Verlauf von 2 Stunden auf 140 °C aufgeheizt und noch weitere 2 Stunden bei dieser Temperatur gerührt. Der Niederschlag ging dabei unter Biuretbildung vollständig in Lösung. Die $CO_2$-Menge erhöhte sich auf korr. 33,8 l (101% d.Th.). Nach Abdestillieren des Pivalinsäureanhydrids bei 20 mbar wurde überschüssiges Hexamethylendiisocyanat durch Dünnschichtdestillation entfernt. Man erhielt 1 180 g eines Biuretpolyisocyanats mit folgenden Eigenschaften:

| | |
|---|---|
| NCO-Gehalt: | 21,4% |
| Viskosität bei 25 °C: | 16 200 mPas |
| APHA-Farbzahl: | 40 |
| Monomerengehalt: | 0,23% |

Beispiel 4

Analog Beispiel 2 wurden aus 4 032 g (24 Mol) Hexamethylendiisocyanat und 408 g (4 Mol) Pivalinsäure 900 g eines dünnflüssigen Biuretpolyisocyanates mit folgenden Eigenschaften erhalten:

| | |
|---|---|
| NCO-Gehalt: | 24,4% |
| Viskosität bei 25°: | 1 250 mPas |
| APHA-Farbzahl: | 35 |
| Monomerengehalt: | 0,15% |

Vergleichsbeispiel I

Analog Beispiel 1 wurden 2 016 g (12 Mol) Hexamethylendiisocyanat vorgelegt und mit 92 g (2 Mol) Ameisensäure versetzt. Die zunächst auf 50 °C ansteigende Temperatur wurde im Verlauf von 2 Stunden auf 120 °C erhöht und noch weitere 2 Stunden bei diesem Wert belassen. Danach war die Reaktion beendet. In einer ersten Gasuhr wurde die Gesamtmenge gemessen, der diese Gasuhr verlassende Strom wurde über zwei Waschflaschen, die mit 20%iger Natronlauge gefüllt waren, in eine zweite Gasuhr geleitet, und so der Kohlenmonoxid-Anteil bestimmt. Es wurde eine Gesamtmenge von korr. 56,7 l (2,53 Mol) gemessen; der Kohlenmonoxidanteil betrug korr. 11,9 l (0,53 Mol). Der NCO-Gehalt der Rohrlösung betrug 40,3%, was einem Verbrauch von 4,76 Mol NCO entspricht. Hieraus, aus der Gasanalyse und aus der Auswertung von gelchromatographischen Analysen ergab sich, dass die Reaktion mit Ameisensäure nur 26,5% Biuretgruppen, aber 73,5% Formylharnstoffgruppen geliefert hatte.

Nach Dünnschichtdestillation erhielt man aus der Rohlösung 650 g eines modifizierten Isocyanats mit folgenden Eigenschaften:

| | |
|---|---|
| NCO-Gehalt: | 22,6% |
| Viskosität bei 25 °C: | 515 mPas |
| APHA-Farbzahl: | 50 |
| Monomerengehalt: | 0,16% |

Das Gelchromatogramm wies als Hauptbestandteil N-Formyl-N,N'-bis(isocyanatohexyl)-harnstoff mit 48,5 Gew.-% aus.

Vergleichsbeispiel II

In diesem Beispiel wurde das Isocyanat bei 120 °C vorgelegt und die Ameisensäure innerhalb 2 Stunden zugetropft. Es wurden folgende Daten erhalten:

| | |
|---|---|
| Gesamtgasmenge: | 3,08 Mol |
| Menge CO: | 1,08 Mol |
| NCO-Gehalt der Rohlösung: | 39,3% |
| NCO-Verbrauch: | 5,43 Mol |
| Biuret-Anteil: | 54% |
| Formylharnstoff-Anteil: | 46% |

Nach Dünnschichtdestillation:

| | |
|---|---|
| Ausbeute: | 750 g |
| NCO-Gehalt: | 22,6% |
| Viskosität bei 25 °C: | 1 200 mPas |
| APHA-Farbzahl: | 70 |
| Monomerengehalt: | 0,23% |
| Gelchromatogramm: | 28,5 Gew.-% des einfachen Formylharnstoffs |

Vergleichsbeispiel III

Hexamethylendiisocyanat wurde bei 160 °C vorgelegt und Ameisensäure in 2 Stunden zugetropft. Es wurden folgende Daten erhalten:

| | |
|---|---|
| Gesamtgasmenge: | 3,44 Mol |
| Menge CO: | 1,44 Mol |
| NCO-Verbrauch: | 6,04 Mol |
| Biuretanteil: | 72% |
| Formylharnstoff-Anteil: | 28% |

daneben aus Gasanalyse, Gelchromatogramm, IR-Spektrum und NCO-Verbrauch 14% Uretdiongruppen.

Nach Dünnschichtdestillation:

| | |
|---|---|
| Ausbeute: | 910 g |
| NCO-Gehalt: | 21,9% |
| Viskosität bei 25 °C: | 1 860 mPas |
| APHA-Farbzahl: | 200 |
| Jodfarbzahl: | 1–2 |
| Monomerengehalt: | 0,3% |
| Gelchromatogramm: | 19,5 |

Gew.-% des einfachen Formylharnstoffs

Vergleichsbeispiel IV

Analog Beispiel 1 wurden 1 008 g (6 Mol) Hexamethylendiisocyanat mit je 2 Mol Essigsäure, Propionsäure und Isobuttersäure umgesetzt.

| | | | |
|---|---|---|---|
| $CO_2$-Menge (Mol) | 1,58 | 1,42 | 1,32 |
| Anhydridausbeute | 42% | 58% | 68% |
| NCO-Gehalt der Rohlösung | 33,6% | 33,1% | 32,5% |
| NCO-Verbrauch (Mol) | 3,58 | 3,42 | 3,32 |
| Jodfarbzahl der Rohlösung | 5–6 | 2–3 | 1–2 |

Wegen der hohen Farbzahlen der Rohprodukte und der niedrigen Anhydridausbeuten wurden die Ansätze nicht aufgearbeitet.

Vergleichsbeispiel V

500 g einer analog Beispiel 1 gewonnenen Biuret-Rohlösung wurden mit 102 g (1 Mol) Acetanhydrid auf 140 °C erhitzt. Nach kurzer Zeit verfärbte sich das Reaktionsgemisch, und eine leichte Gasentwicklung setzte ein. Nach 3 Stunden waren 13% des eingesetzten Anhydrids verbraucht. Nach weiteren 2 Stunden bei 160° betrug der Anhydridverbrauch 53,3%. Das Gemisch war tiefrot gefärbt.

Setzte man anstelle des Acetanhydrids die äquivalente Menge Pivalinsäureanhydrid ein, so war nach der gleichen Temperaturbehandlung keine Veränderung des Reaktionsgemisches festzustellen; das Anhydrid konnte danach quantitativ abdestilliert werden.

Beispiel 5

Analog Beispiel 2 wurden 630 g (3 Mol) Trimethyl-hexandiisocyanat (Isomerengemisch 2,24- und 2,4,4-) mit 126 g (1 Mol) 2,2,3-Trimethylbuttersäure umgesetzt. Es wurde die berechnete Menge (0,5 Mol) $CO_2$ freigesetzt, bei 0,1 mb wurden 109 g des entsprechenden Säureanhydrids abdestilliert (kp: 60–64°).

Nach Dünnschichtdestillation wurden 250 g eines viskosen Biuretpolyisocyanats mit folgenden Eigenschaften erhalten:

| | |
|---|---|
| NCO-Gehalt: | 18,3% |
| Viskosität bei 25°: | 97 500 |
| APHA-Farbzahl: | 30 |
| Monomerengehalt: | 0,4% |

Beispiel 6

2 016 g (12 Mol) 3-Methylpentan-1,5-diisocyanat wurden bei 80° vorgelegt. Aus zwei getrennten Tropftrichtern wurden im Verlauf von 2 Stunden synchron 200 g (2 Mol) 1-Methylcyclopropan-carbonsäure mit 18 g (1 Mol) dest. Wasser zugetropft.

Nach beendeter Zugabe wurde noch 30 Min. bei 100° und 30 Min. bei 120° nachgerührt. Danach war die $CO_2$-Entwicklung beendet. Nun wurde bei 2 mbar eine Fraktion von 320 g abdestilliert. Diese bestand nach GC und NCO-Bestimmung zu 52,9% aus dem Säureanhydrid (93% der Theorie) und zu 47,0% aus dem eingesetzten Diisocyanat.

Nach Dünnschichtdestillation erhielt man 810 g eines Biuretpolyisocyanats mit folgenden Eigenschaften:

| | |
|---|---|
| NCO-Gehalt: | 22,5% |
| Viskosität bei 25°: | 31 900 mPas |
| APHA-Farbzahl: | 20–30 |
| Monomerengehalt: | 0,3% |

Beispiel 7

6 048 g (36 Mol) Hexamethylendiisocyanat und 300 ml 1,4-Dioxan wurden bei 100° vorgelegt und eine Lösung von 155 g (1,33 Mol) 2,2-Dimethylbuttersäure und 60 g (3,3 Mol) dest. Wasser in 200 ml 1,4-Dioxan im Verlauf von 3 Stunden zugetropft. Nach einer weiteren Stunde bei 130° war die Gasentwicklung (4 Mol) beendet. Nun wurde bei 50 mbar zunächst das Lösungsmittel abgezogen und anschliessend bei 15 mbar eine Fraktion (200 g) abdestilliert, die nach Redestillation 123 g des reinen Säureanhydrids (Kp: 118°/15 mbar) ergab (94% der Theorie). Die nachfolgende Dünnschichtdestillation ergab 1 730 g eines niedrigviskosen Biuretpolyisocyanats mit folgenden Eigenschaften:

| | |
|---|---|
| NCO-Gehalt: | 23,6% |
| Viskosität bei 25°: | 2 350 mPas |
| APHA-Farbzahl: | 30 |
| Monomerengehalt: | 0,18% |

## Patentansprüche

1. Verfahren zur Herstellung von Polyisocyanaten mit Biuretstruktur durch Umsetzung von überschüssigen Mengen an aliphatischen Diisocyanaten mit Biuretisierungsmitteln bei erhöhter Temperatur, gegebenenfalls unter Überdruck, wobei die Umsetzung gegebenenfalls in Gegenwart von mit Wasser mischbaren Lösungsmitteln erfolgt, und wobei gegebenenfalls im Anschluss an die Biuretisierungsreaktion überschüssige Mengen an nicht umgesetztem Ausgangsdiisocyanat, sowie Lösungsmittel und sonstige flüchtige Bestandteile des Reaktionsgemisches durch Destillation und/oder Extraktion von dem entstandenen Biuretpolyisocyanat abgetrennt werden, dadurch gekennzeichnet, dass man als Biuretisierungsmittel

a) α, α,α-trisubstituierte Essigsäuren, welche neben der Carboxylgruppe keine gegenüber Isocyanatgruppen reaktionsfähige Gruppen aufweisen und gegebenenfalls

b) Wasser verwendet, wobei ein Molverhältnis der Biuretisierungsmittelkomponenten a) zu b) von 1:0 bis 1:2,5 eingehalten wird.

2. Verfahren gemäss Anspruch 1, dadurch gekennzeichnet, dass als aliphatisches Diisocyanat 1,6-Diisocyanatohexan eingesetzt wird.

3. Verfahren gemäss Anspruch 1 und 2, dadurch gekennzeichnet, dass als α,α,α-trisubstituierte Essigsäuren solche der allgemeinen Formel

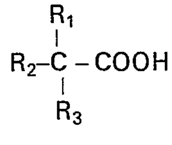

verwendet werden,

in der $R_1$, $R_2$ und $R_3$ für gleiche oder verschiedene Reste stehen und Alkyl-, Alkoxy- oder Alkoxyalkyl-Gruppen bedeuten, wobei gegebenenfalls zwei der Reste zusammen mit dem substituierten Kohlenstoffatom der Essigsäure einen cycloaliphatischen Ring bilden.

4. Verfahren gemäss Anspruch 1 bis 3, dadurch gekennzeichnet, dass man als α,α,α-trisubstituierte Essigsäure Trimethylessigsäure (Pivalinsäure) verwendet.

## Revendications

1. Procédé de production de polyisocyanates à structure de biuret par réaction de quantités en excès de diisocyanates aliphatiques avec des agents de formation de biuret à température élvée, le cas échéant en surpression, en conduisant éventuellement la réaction en présence de solvants miscibles à l'eau et en séparant le cas échéant, à la suite de la réaction de formation de biuret, les quantités en excès de diisocyanate de départ n'ayant par réagi ainsi que le solvant et d'autres constituants volatils du mélange réactionnel par distillation et/ou par extraction du biuret-polyisocyanate formé, caractérisé en ce qu'on utilise comme agents de formation de biuret

a) des acides acétiques α,α,α-trisubstitués qui, à côté du groupe carboxyle, ne présentent aucun groupe apte à réagir vis-à-vis de groupes isocyanato et le cas échéant

b) de l'eau en maintenant le rapport molaire du composant a) au composant b) de l'agent de formation de biuret de 1:0 à 1:2,5.

2. Procédé suivant la revendication 1, caracté risé en ce qu'on utilise comme diisocyanate aliphatique le 1,6-diisocyanatohexane.

3. Procédé suivant les revendications 1 et 2, caractérisé en ce qu'on utilise comme acides acétiques α,α,α-trisubstitués les acides de formule générale

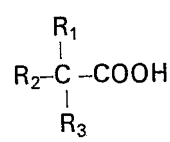

dans laquelle $R_1$, $R_2$ et $R_3$ sont des restes identiques ou différents et représentent des groupes alkyle, alkoxy ou alkoxyalkyle, deux des restes formant le cas échéant un noyau cycloaliphatique conjointement avec l'atome substitué de carbone de l'acide acétique.

4. Procédé suivant les revendications 1 à 3, caractérisé en ce qu'on utilise comme acide acétique α,α,α-trisubstitué l'acide triméthylacétique (acide pivalique).

## Claims

1. Process for the preparation of polyisocyanates having a biuret structure by the reaction of excess quantities of aliphatic diisocyanates with biuretizing agents at elevated temperatures, optionally under excess pressure, the reaction being optionally carried out in the presence of water-miscible solvents, and excess quantities of unreacted starting diisocyanate and solvent and other volatile constituents of the reaction mixture being optionally separated from the resulting biuret polyisocyanate by distillation and/or extraction after the biuretization reaction, characterised in that the biuretizing agents used are

a) α,α,α-trisubstituted acetic acids which do not contain any isocyanate reactive groups apart from the carboxyl group and optionally

b) water, a molar ratio of biuretizing agent component a) to b) of from 1:0 to 1:2,5 being maintained.

2. Process according to claim 1, characterised in that the aliphatic diisocyanate used is 1,6-diisocyanatohexane.

3. Process according to claims 1 and 2, characterised in that the α,α,α-trisubstituted acetic acids used are compounds corresponding to the following general formula

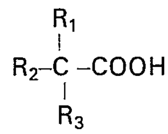

wherein $R_1$, $R_2$ and $R_3$ may be identical or different and stand for alkyl, alkoxy or alkoxyalkyl groups, two of the groups optionally combining with the substituted carbon atom of the acetic acid to form a cycloaliphatic ring.

4. Process according to claims 1 to 3, characterised in that the α,α,α-trisubstituted acetic acid used is trimethylacetic acid (pivalic acid).